# EUROPEAN PATENT SPECIFICATION

(11) **EP 3 725 354 B1**
(45) Date of publication and mention of the grant of the patent: **12.04.2023**
(21) Application number: 19181675.0
(22) Date of filing: 21.06.2019
(51) Int. Cl.: A61M 21/00, G06F 3/01, G16H 20/30, G16H 40/63, G06F 3/16, G06N 3/04, G06N 3/08, G16H 50/20, A63F 13/211, A63F 13/25, A63F 13/428, A63F 13/5255, A63F 13/54

(54) **AUDIO CONTROLLER**
AUDIOSTEUERGERÄT
ORGANE DE COMMANDE AUDIO

(30) Priority: 15.04.2019 IN 201911014990
(43) Date of publication of application: 21.10.2020
(73) Proprietor: NXP B.V., 5656 AG Eindhoven (NL)
(72) Inventor: SHEKAR, Pramod Rajan Kesavelu, Redhill Surrey RH1 1QZ (GB); MEIJER, Rinze Ida Mechtildis Peter, Redhill Surrey RH1 1QZ (GB)
(74) Representative: Miles, John Richard

(56) References cited:
- US-A1- 2015 325 027
- US-A1- 2016 228 771
- US-A1- 2017 253 252
- US-A1- 2018 096 517
- US-A1- 2018 221 621

## Description

### FIELD

This disclosure relates to a method and apparatus for controlling audio signal generation for prevention of motion sickness.

### BACKGROUND

Motion sickness is caused by movement and its relationship to the inner ear and balance. Depending on the cause, it is typically referred to as car sickness, sea sickness, or air sickness. When travelling by car, drivers are less likely to be affected by motion sickness compared to passengers. Anyone can develop motion sickness, but people vary in their sensitivity to motion. Symptoms of motion sickness are: nausea, vomiting, dizziness, sweating, and a sense of feeling unwell. These symptoms arise from the inner ear due to changes in a person's sense of balance and equilibrium. Virtual Reality (VR) sickness occurs when exposure to a virtual environment causes symptoms similar to motion sickness symptoms. VR sickness is different from motion sickness in that it can be caused by the visually-induced perception of self-motion; real self-motion is not needed. This can be a problem in particular with any games which create a significant perceived mismatch between actual and virtual body position.

It is known that infrasonic sounds may have adverse effects such as nausea, dizziness and headaches. Other audio tones having certain frequencies which may be audible or inaudible and pulse rates may desensitize the Vestibular system providing a therapeutic effect which may alleviate or prevent motion sickness symptoms. An example of such a system is described in US6228021B1.

US 2018/0096517 A1 describes a system for field of view throttling of virtual reality content in a head-mounted display.

US 2016/228771 A1 describes motion sickness monitoring an application of supplemental sound to counteract sickness.

US 2015/325027 A1 show a system for reducing motion sickness in virtual reality ride systems. US 2018/221621 A1 shows an apparatus that outputs a sound effect reducing sickness of a user viewing a video. US 2017/253252 A1 shows a sensory stimulation system that provides sensory stimulation outputs responsive to or preemptive of maneuvers by an autonomous vehicle.

### SUMMARY

The present invention is defined by the appended claims. Aspects, embodiments or examples of the present disclosure which do not fall within the scope of the appended claims do not form part of the present invention and are present for illustrative purposes. Methods disclosed hereinafter are also not part of the present invention and are present for illustrative purposes.

The present disclosure relates to an audio controller for generating a signal for alleviating motion sickness, the audio controller comprising: a sensor input module; a user control input; a processor comprising a machine learning model corresponding to a desired audio stimulation profile, the machine learning model being coupled to the sensor input module and the user control input and configured to receive a sensor signal comprising at least one user attribute and at least one context attribute from the sensor input module; a stimulus generator coupled to the machine learning model; and wherein the machine learning model is configured to control the stimulus generator to generate a reference signal for alleviating motion sickness, the reference signal being adapted dependent on at least one of the user control input, and the sensor signal.

In one or more embodiments, the machine learning model may comprise a neural network and the processor is configured to adapt the machine learning model by modifying the activation threshold of layers of the neural network dependent on the sensor signal.

In one or more embodiments, the machine learning model may comprise a first neural network configured to control the period between bursts of the audio stimulus, a second neural network configured to control the gain of the audio stimulus, and a third neural network configured to control the offset of the audio stimulus.

In one or more embodiments, the processor may be configured to compile additional training data dependent on the external user input and the sensor signal; to adapt the machine learning model in a training cycle using training data comprising the additional training data and to generate an audio stimulation profile comprising the adapted machine learning model.

In one or more embodiments, the audio controller may further comprise a memory coupled to the processor and configured to update the audio stimulation profiles in the memory with the adapted machine learning models.

In one or more embodiments, the audio controller may further comprise an audio input configured to receive an audio signal; a mixer coupled to the audio input and the stimulus generator output; wherein the processor comprises a further machine learning model corresponding to the desired audio stimulation profile coupled to the audio input and a filter.

The further machine learning model may be configured to control the filter to adapt the audio signal to at least attenuate infrasound signals dependent on at least one of an external user input and the sensor signal; and the mixer may be further configured to mix the adapted audio signal and the reference signal and to output the mixed adapted audio signal and the reference signal. In one or more embodiments, the desired audio stimulation profile may be determined from at least one of a user control input and the sensor signal.

In one or more embodiments, the sensor input module may be configured to receive a video frame rate from a video device and wherein the at least one context attribute comprises the video frame rate.

In one or more embodiments, the sensor input module may be configured to receive at least one of a tilt value and a user seating position value, and wherein the at least one context attribute comprises at least one of the speed of a vehicle, the tilt of a vehicle, and the user seating position and the at least one user attribute comprises at least one of head motion and head tilt. Embodiments of the audio controller may be included in a wearable device. The wearable device may further comprise a wireless receiver coupled to the sensor input, wherein the wearable device is configured to receive context attributes via the wireless receiver.

The present disclosure also relates to a computer program product comprising instructions which, when being executed by a processing unit, cause said processing unit to perform the steps of receiving a sensor signal comprising at least one user attribute and at least one context attribute from the sensor input module; providing the sensor signal and the at least one context attribute to a machine learning model; controlling a stimulus generator using the machine learning model to generate a reference signal dependent on at least one of the adapted audio stimulation profile and the user input.

In one or more embodiments, the machine learning model comprises a neural network and the processing unit may perform the steps of adapting the machine learning model by modifying the activation threshold of layers of the neural network dependent on the sensor signal.

In one or more embodiments, the processing unit may perform the steps of controlling the period between bursts of the audio stimulus with a first neural network of the machine learning model, controlling the gain of the audio stimulus with a second neural network of the machine learning model, and controlling the offset of the audio stimulus with a third neural network of the machine learning model.

In one or more embodiments, the processing unit may perform the steps of compiling additional training data dependent on the external user input and the sensor signal; adapting the machine learning model in a training cycle using training data comprising the additional training data; and storing the adapted machine learning model.

In one or more embodiments, the processing unit may perform the steps of: receiving an audio signal; using a further machine learning model corresponding to the desired audio stimulation profile to control the filtering of the audio signal to at least attenuate infrasound signals dependent on at least one of the external user input and the sensor signal; mixing the filtered audio signal and the reference signal.

### BRIEF DESCRIPTION OF THE DRAWINGS

In the figures and description like reference numerals refer to like features. Embodiments of are now described in detail, by way of example only, illustrated by the accompanying drawings in which:
Figure 1 shows an example audio controller for generating a signal to alleviate motion sickness.
Figure 2 illustrates an example audio controller for generating a signal to alleviate motion sickness.
Figure 3 shows a method for generating a signal to alleviate motion sickness.
Figure 4 shows a method for generating a signal to alleviate motion sickness.
Figure 5 shows an audio controller for generating a signal to alleviate motion sickness.
Figure 6 shows an audio controller for generating a signal to alleviate motion sickness in a vehicle.
Figure 7 shows an example stimulus generation adaptation module.
Figure 8 shows an example audio mixing adaptation module.
Figure 9 shows an example intelligently controlled auditory device including an audio controller for generating a signal to alleviate motion sickness.
Figure 10 shows the intelligently controlled auditory device of Figure 9 in a vehicle context.
Figure 11 shows the intelligently controlled auditory device of Figure 9 used with a mobile device.
Figure 12 shows the intelligently controlled auditory device of Figure 9 in a computer game context.

### DETAILED DESCRIPTION

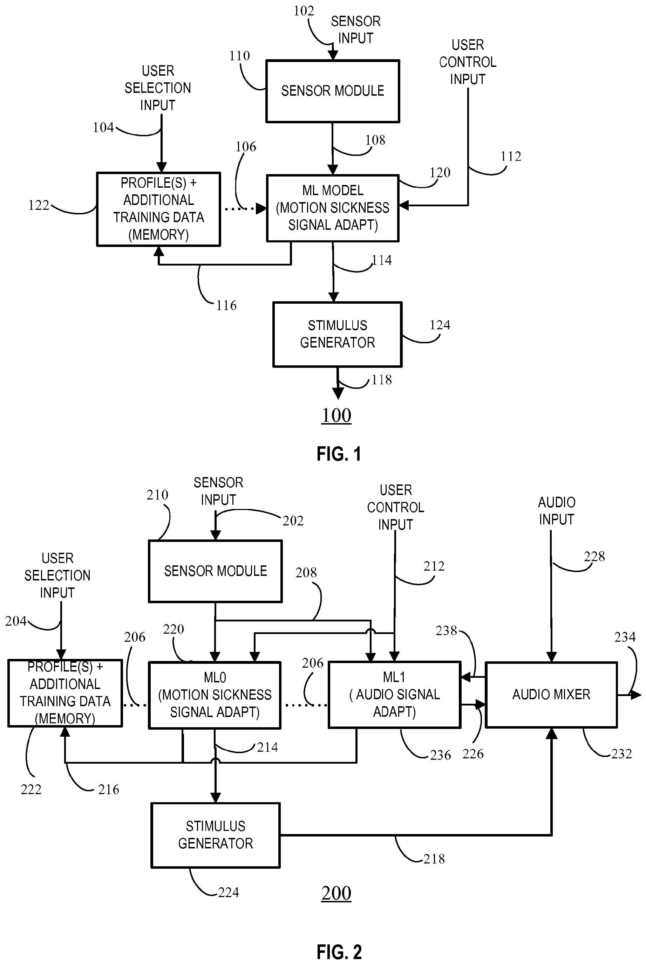

Figure 1 shows an audio controller 100. A sensor module 110 may have a sensor input 102. The sensor module 110 may have a sensor output 108 coupled to a first input of a machine learning model 120. The machine learning model 120 may have a second input connected to a user control input 112. The machine learning model 120 may have a third input connected to an output 106 of a memory or storage element 122. The machine learning model 120 may have a first output 114 connected to an input of a stimulus generator 124. The machine learning model 120 may have a second output 116 connected to a first input of the memory 122. The memory 122 may a second input connected to a user selection input 104. The stimulus generator 124 may have a stimulus generator output 118.

In operation the sensor input 102, may be connected to one or more sensors. The connection to the sensors may vary dependent on the context of the audio controller 102. For example, in some examples the audio controller 100 may be integrated into a hearable device or other wearable device. If the user is in the car, the hearable device may communicate wirelessly with the sensor data via a communication link between the hearable device and the car by using an RF connection such as Bluetooth or Near Field Magnetic Induction (NFMI). Alternatively, or in addition, in other examples the sensor input 102 may have a wired connection to one or more sensors included in the hearable device. This sensor data may include context attribute data such as the speed of the vehicle, the tilt of the vehicle, and the altitude. In other examples, if the user is using a VR headset, the VR headset may communicate with a hearable and provide sensor data such as video frame rate and scene information from. In other examples, the audio controller 100 may be included for example in a car infotainment system and VR headset. In addition, the sensor input 102 may be connected to sensors that provide user attribute data. This user attribute data may include information such as head motion, head tilt, other body motion such as jumping and physiological measurements such as heart rate and body temperature. The sensor module 110 may receive sensor inputs combine the data and output the sensor data on the sensor module output 108 to the machine learning model 120.

The memory 122 may include one or more profiles each corresponding to a configuration of the machine learning model 120. The term machine learning model 120 in this application may be considered as a model typically implemented as a neural network which has been configured or trained using some training data. The machine learning model may be implemented in software executable by for example a microprocessor, graphics processor, or digital signal processor. In other examples the processor may be a dedicated Artificial Intelligence (AI) processor which includes hardware circuits configurable as a neural network. In examples where the machine learning model is implemented in hardware in the processor, the processor may be considered to be an inference engine. The memory 122 may also include additional training data or a history which may be used to further adapt the machine learning model 120. This training data typically includes context attribute data, user attribute data and a label of the desired response of the machine learning model to the context attribute data and user attribute data. The desired response may be control values for stimulus amplitude, burst period, burst frequency, combination of tones used. In some examples the memory may be considered to be part of the processor. In this case, the separate memory 122 may be omitted.

In operation, on initialization, a user of the audio controller 100 may select the profile for the machine learning model to use via the user selection input 104. This selection may be determined from one or more context attributes such as whether the user us in a car, aeroplane, train, boat or whether the user is using a VR headset. Alternatively, the machine learning model 120 may request a profile based on sensor inputs received from the sensor module 110.

The machine learning model 120 may control the stimulus generator 124 to generate an audio signal for alleviating motion sickness dependent on the sensor inputs received from the sensor module 108. The machine learning model 120 may generate control signals sent to the stimulus generator 124 which adapt characteristics of the generated reference signal such as burst frequency, period and a strength or amplitude. The user may apply an additional control signal via user control input 112 to adjust the characteristics of the audio stimulus while the audio controller 100 is in operation. These characteristics may include a gain, a period between each stimulus burst, or duration of each stimulus burst of the stimulus generation. This may be done for example by overriding the output of the machine learning model 120. In other examples, the weights applied to one or more layers of the neural network of the machine learning model 120 may be adjusted by using the additional input as training data to further adapt the machine learning model 120. In either case the user input may be captured along with the inputs of the sensor module to provide additional training data which may subsequently be stored in the memory 122.

In other examples, the machine learning model 120 may be directly adapted if the sensor module indicates conditions that are known to be highly likely to cause motion sickness, for example in the case of a car, if the sensors indicate frequent changes of direction above a certain threshold, the threshold of the activation function of one or more layers of the neural network of the machine learning model 120 may be adjusted to indicate an increase in the strength of the stimulus to the stimulus generator 124.

The inventors of the present disclosure have appreciated that by applying machine learning to the auditory stimulus, the audio controller 100 may automatically adapt the generated stimulus dependent on the specific preferences of the user and so implement an intelligently controlled auditory device. Further, in scenarios where the user wishes to adjust the automatically generated stimulus, the audio controller 100 may use the captured additional user preferences together with the sensor inputs at the time to initiate one or more further training cycles to further adapt the machine learning model 120. These additional training cycles may be initiated during a standby mode of the audio controller 100, on initialization or power up, or during some other suitable time. The profiles corresponding to the modified machine learning model configurations may be stored in memory for subsequent use by the audio controller 100.

Figure 2 shows an audio controller 200. A sensor module 210 may have a sensor input 202. The sensor module 210 may have a sensor output 208 coupled to a first input of a motion sickness adaptor machine learning model 220. The motion sickness adaptor machine learning model 220 may have a second input connected to a user control input 212. The motion sickness adaptor machine learning model 220 may have a third input connected an output 206 of a memory or storage element 222. The motion sickness adaptor machine learning model 220 may have a first output 214 connected to an input of a stimulus generator 224. The motion sickness adaptor machine learning model 220 may have a second output connected to a first input 216 of the memory 222.

The sensor output 208 may be connected to a first input of an audio mixer machine learning model 236. The audio mixer machine learning model 236 may have a second input connected to a user control input 212. The audio mixer machine learning model 236 may have a third input connected to an output 206 of a memory or storage element 222. The audio mixer machine learning model 236 may have a first output 226 connected to a third input of the audio mixer 232. The audio mixer machine learning model 236 may have a second output 216 connected to a first input 216 of the memory 222.

The memory 222 may have a second input connected to a user selection input 204. The stimulus generator 224 may have a stimulus generator output 218 connected to first input of an audio mixer 232. An audio input 228 may be connected to a second input of audio mixer 232. The audio mixer 232 may have a first audio output 234. The audio mixer 232 may have a second audio output 238.

In operation the sensor input 202, may be connected to one or more sensors. The connection to the sensors may vary dependent on the context of the audio controller 200. For example, in some examples the audio controller 200 may be integrated into a hearable device, other wearable device or mobile device. The sensor data received on the sensor input 202 may include context attribute data such as the speed of the vehicle, the tilt of the vehicle, and the altitude. In other examples, if the user is using a VR headset, the VR headset may communicate with a hearable and provide sensor data such as video frame rate and scene information from a video game. In other examples, the audio controller 200 may be included for example in a car infotainment system or other vehicle electronic system, or a VR headset. In addition, the sensor input 202 may be connected to sensors that provide user attribute data. This user attribute data may include information such as head motion, head tilt, other body motion such as jumping and physiological measurements such as heart rate and body temperature. The sensor module 210 may receive sensor inputs and combine the received sensor data. The sensor module 210 may output the sensor data on the sensor module output 208 to the motion sickness adaptor machine learning model 220 and the audio mixer machine learning model 236.

The memory 222 may include one or more profiles each corresponding to a configuration of each of the motion sickness adaptation machine learning model 220 and the audio mixer machine learning model 236. The term machine learning model as used in the present disclosure may be considered as a neural network which has been configured or trained using some training data. The machine learning model may be implemented as software running on a microprocessor, graphics processor or using a more dedicated Artificial Intelligence (Al) processor which has an architecture to support the implementation of neural networks. The memory 222 may also include additional training data or a history which may be used to further adapt each of the motion sickness adaptation machine learning model 220 and the audio mixer machine learning model 236. For the motion sickness adaptation machine learning model 220, this training data typically includes context attribute data, user attribute data and the desired response of the machine learning model to the context attribute data and user attribute data. The desired response may include control values for stimulus amplitude, burst period, burst frequency, combination of tones used. For the audio mixer machine learning model 236, this training data typically includes context attribute data, user attribute data and the desired response of the machine learning model to the context attribute data and user attribute data. The desired response may be for example control values for the audio mixer 232.

In operation, on initialization, a user of the audio controller 200 may select the profile for each machine learning model 220, 236 to use via the user selection input 204. This selection may be determined from one or more context attributes such as whether the user is in a car, aeroplane, train, boat or whether the user is using a VR headset. Alternatively, the machine learning model 220 may request a profile based on sensor inputs received from the sensor module 210.

The motion sickness adaptation machine learning model 220 may control the stimulus generator 224 to generate an audio signal for alleviating motion sickness dependent on the sensor inputs received from the sensor module 210. The machine learning model 220 may generate control signals sent to the stimulus generator 224 which adapt characteristics of the generated signal such as burst frequency, period and a strength or amplitude. The user may apply an additional control signal via user control input 212 to adjust the characteristics of the audio stimulus while the audio controller 200 is in operation.

This may be done for example by overriding the output of the motion sickness adaptation machine learning model 220. In other examples, the weights applied to one or more layers of the neural network of the motion sickness adaptation machine learning model 220 may be adjusted by using the additional input as training data to further adapt the machine learning model 220. In either case the user input may be captured along with the inputs of the sensor module to provide additional training data which may subsequently be stored in the memory 222.

In other examples, the motion sickness adaptation machine learning model 220 may be directly adapted by adjusting the threshold of the activation function, if the sensor module indicates conditions that are known to be highly likely to cause motion sickness. For example, in the case of a car, if the sensors indicate frequent changes of direction above a certain threshold, the threshold applied to the activation function of the neural network of the motion sickness adaptation machine learning model 220 may be adjusted to indicate an increase in the strength of the stimulus to the stimulus generator 224. The activation function used may be a ReLu (rectified linear units) activation function. It can be described as follows: R(x) = max (0, x) i.e. if x < 0, R(x) = 0 and if x >= 0, R(x) = x. Such function may be implemented very efficiently. Other examples may use other activation functions such as Sigmoid or Tanh.

The audio mixer machine learning model 236 may control the audio mixer 232 to adapt the audio signal received on the audio input 228. The audio mixer machine learning model 236 may control the mixing of an audio signal received on the audio input 228 together with the output of the stimulus generator for alleviating motion sickness dependent on the sensor inputs received from the sensor module 210.

The audio mixer machine learning model 236 may receive an audio signal from the audio mixer 232 on the audio mixer machine learning model output 238. The audio mixer machine learning model 236 may generate control signals sent to the audio mixer 232 by first machine learning model output 226 which adapt characteristics of the received audio signal such as filter settings and gain strength to filter out infrasound and other frequencies known to have adverse effects such as nausea, dizziness and headaches. The user may apply an additional control signal via user control input 212 to adjust the characteristics of the audio input signal while the audio controller 200 is in operation.

This may be done for example be overriding the output of the audio mixer machine learning model 236. In other examples, the weights applied to one or more layers of the neural network of the audio mixer machine learning model 236 may be adjusted by using the additional input as training data to further adapt audio mixer machine learning model 236. In either case the user input may be captured along with the inputs of the sensor module to provide additional training data which may subsequently be stored in the memory 222.

The audio controller 200 may automatically adapt the generated stimulus for reducing motion sickness together with any other audio input stimulus dependent on the specific preferences of the user and so implement an intelligently controlled auditory device. By generating an audio input stimulus which may reduce motion sickness and filtering out audio frequencies, for example infrasound frequencies which potentially cause nausea and dizziness and headaches, the audio controller 200 may improve the well-being of a user. Further, in scenarios where the user wishes to adjust the automatically generated stimulus, the audio controller 200 may use the captured additional user preferences together with the sensor inputs at the time to initiate one or more further training cycles to further adapt the machine learning models 220,236.

These additional training cycles may be initiated during a standby mode of the audio controller 200, on initialization or power up, or during some other suitable time. The profiles corresponding to the modified machine learning model configurations may be stored in memory for subsequent use by the audio controller 200. The machine learning models 220, 236 may be trained independently from each other.

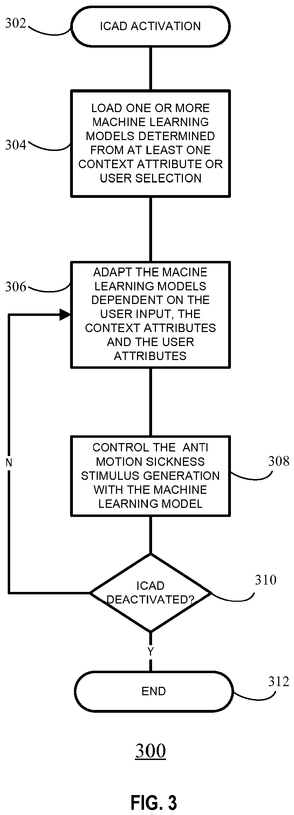

Figure 3 shows a method of operating an intelligently controlled auditory device (ICAD) for alleviating motion sickness 300. In step 302 the ICAD device may be activated. In step 304 default profiles may be loaded into a respective machine learning model determined from at least one context attribute or user selection. If a machine learning model configuration or profile is stored in local non-volatile memory, then step 304 may be omitted. The context attribute may be provided from a sensor input which may indicate for example that the user is in either a car, an aeroplane, or a boat. In step 306 the profiles may be further adapted for each machine learning model dependent on one or more of the user input, the context attributes and the user attributes. The adaptation may be done by training the machine learning model with updated training data or direct adaptation from the user input or context and user attributes as described for audio controllers 100 and audio controller 200. In step 308 the outputs of the machine learning models may control the generation based on the adapted profiles. In step 310 the method may check whether a deactivation signal has been received. If a deactivation signal has been received, the method proceeds to step 312 and finished. Returning to step 310 if the device has not been deactivated, the method returns to the adaptation step 306.

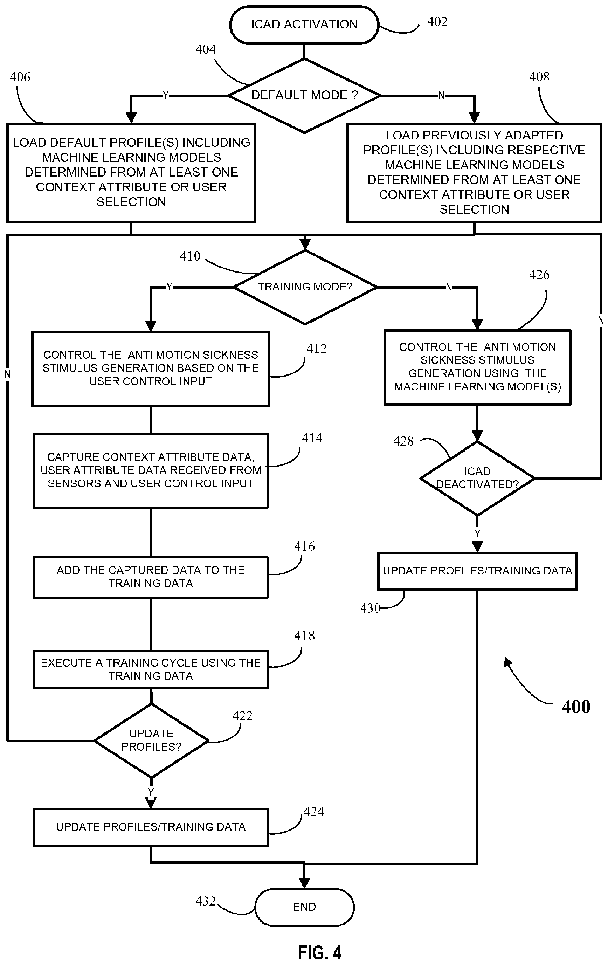

Figure 4 shows a method controlling an auditory device 400. In step 402 the auditory device may be activated 402. In step 404, the method may check whether a default mode is required. If the mode is the default mode, then the method proceeds to step 406 and default profiles maybe added into respective machine learning models. If a machine learning model configuration or profile is stored in local non-volatile memory, then step 406 may be omitted. The default profile may be determined either directly from a user input or from at least one context attribute which may for example received from a sensor. The context attribute may determine where a user is located, for example, in a vehicle, boat, aeroplane, the context attribute may determine whether the user is using a VR headset or is in a simulator such as a flight simulator. Returning to step 404, if the mode is not a default mode, the method proceeds to step 408 and a previously adapted profile or profiles is loaded to configure one or more machine learning models. If a machine learning model configuration or profile is stored in local non-volatile memory, then step 408 may be omitted. The particular adapted profile may be similarly determined as done in step 406. Following from steps 406 or 408, the method proceeds to step 410 where a determination is made as to whether the audio controller is in a training mode. If the audio controller is not in a training mode, the method proceeds to step 426. The anti-motion sickness stimulus generation is controlled using one or more machine learning models as previously described for audio controller 100 and audio controller 200. Following on from step 426, the method proceeds to step 428 to determine whether or not the audio controller has received a deactivation signal. If the audio controller has received a deactivation signal, the method proceeds to step 430 in which any training data or updated profiles may be stored. Following step 430 the method 400 terminates at step 432. Returning to step 428, if the audio controller has not received a deactivation signal, the method returns to step 410.

Returning to step 410, if the audio controller is in training mode, the method moves to step 412. The anti-motion sickness stimulus generation is controlled using one or more machine learning models as previously described for audio controller 100 and audio controller 200. In step 414 the context attribute data, the user attribute data received from one or more sensors and a user control input may be captured. In step 416, the captured data may be added to the training data 416. In step 418 a training cycle may be executed using the training data. The training cycle typically applies the training data to the neural network of the machine learning model over a number of epochs and may result in a new configuration of the machine learning model or machine learning models. In step 422 the method may determine whether or not machine learning model configurations or profiles may be updated. If the audio controller has received a deactivation signal, the method proceeds to step 424 in which any training data or updated profiles may be stored. Following step 424 the method 400 terminates at step 432. Returning to step 422, if the audio controller has not received a deactivation signal, the method returns to step 410.

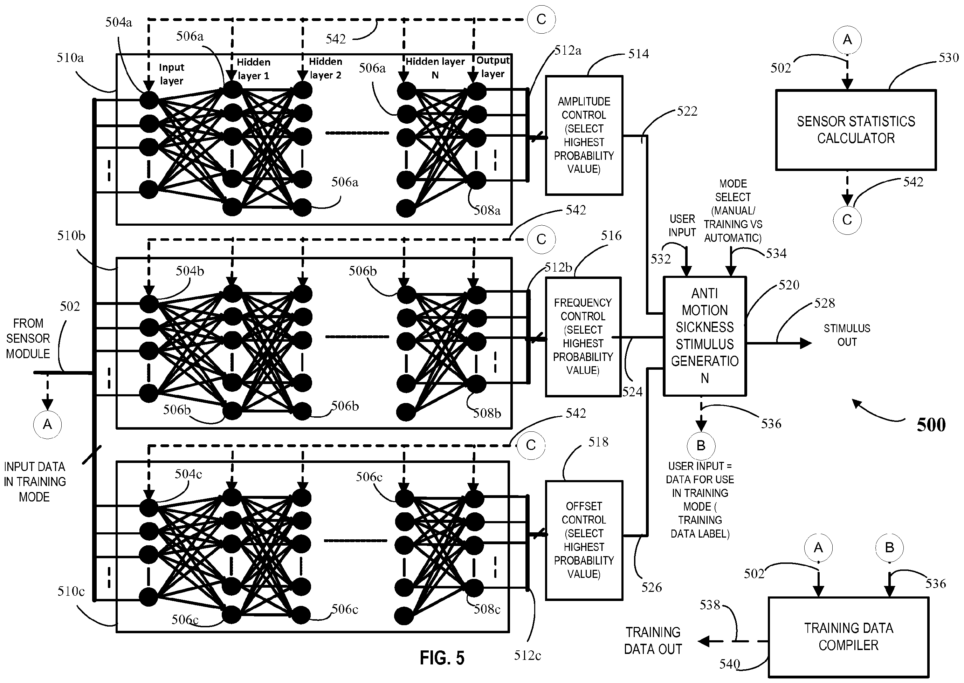

Figure 5 shows an example of an audio controller including an artificial intelligence (Al) processor 500. AI processor 500 may include a number of machine learning models 510a, 510b, 510c. Each of the machine learning models 510a, 510b, 510c may adapt a specific parameter used as in put the control the anti-motion sickness stimulus generation implemented by stimulus generator 520.

Each machine learning model 510a, 510b, 510c may be implemented as a neural network having a respective input layer 504a, 504b, 504c, a respective number of hidden layers 506a, 506b, 506c and an output layer 508a, 508b, 508c. The number of hidden layers 506a, 506b, 506c may be different in each of the machine learning models 510a, 510b, 510c. the AI processor 500 may have a processor input 502 (node A) which may be a bus connected to each of the input layers 504a, 504b, 504c. The output 512a of the first machine learning model 510a may be connected to a gain controller 514. The output 512b of the second machine learning model 510b may be connected to a frequency controller 516. The output 512c of the third machine learning model 510c may be connected to an offset controller 518.

The gain controller output 522 may be connected to a first input of the stimulus generator 520. The frequency controller output 524 may be connected to a second input of the stimulus generator 520. The offset controller output 526 may be connected to a third input of the stimulus generator 520. The stimulus generator 520 may have a user input 532 and a mode select input 534. The stimulus generator 520 may have a stimulus output 528 and a user control output 536 (node B) connected to a first input of a training data compiler 540. A second input of the training data compiler 540 may be connected to the processor input 502. Training data compiler 540 may have a training data output 538.

A sensor statistics calculator 530 may have an input connected to the processor input 502. The sensor statistics calculator 530 may have a sensor statistics calculator output 542 (node C) connected to the respective layers of the machine learning models 51 0a, 51 0b, 510c.

In this example the AI processor 500 has a machine learning model 510a, 510b, 510c for each of the control parameters. An initial profile may be configured from a learning cycle using offline data consisting of a test set of sensor data and expected responses for each of the control parameters. The expected responses are the required amplitude setting, the required burst frequency, that is to say the period between each burst and the required offset which may be used to increase or decrease the amplitude of each burst by a fixed amount. Each machine learning model may have multiple outputs, each output corresponding to a specific setting for each control parameter. As will be understood, the neural network may be trained to classify which control parameter setting is the most desirable i.e. has the highest probability from the training data by minimising the loss function using the training data. This initial training is done off line and the resulting trained neural networks may implement the machine learning models 510a, 510b and 510c.

In operation, the initial profile or machine learning model configurations may be selected either based on sensor input information received on the processor input 502 or by user selection. The sensor input 502 may be connected to sensors that provide user attribute data or context attribute data. This user attribute data may include information such as head motion, head tilt, other body motion such as jumping and physiological measurements such as heart rate and body temperature. The context attribute data may include data such as the speed of the vehicle, the tilt of the vehicle, and the altitude.

Each machine learning model 510a, 510b and 510c may receive sensor input data and output respectively a required amplitude control, frequency control and offset control. In this example three machine learning models are shown, but it will be appreciated that the number of inference engines may vary depend on the number of different features from the sensor input data.

The gain controller 514 may generate a control signal for the stimulus generator 520 corresponding to the required amplitude of the auditory stimulus. The frequency controller 516 may generate a control signal for the stimulus generator 520 corresponding to the required burst frequency of the auditory stimulus. The offset controller 518 may generate a control signal for the stimulus generator 520 corresponding to the required offset of the auditory stimulus. The stimulus generator 520 may generate an auditory stimulus determined from the amplitude, frequency and offset.

The machine learning models 510a, 5 10b, 510c may be further adapted by the sensor statistics calculator 530. This may adapt the activation thresholds of the layers from a-priori rules on characteristics which may increase or reduce the likelihood of motion sickness. For example, if the sensor data indicates frequent change of direction or variation of tilt of a vehicle above a predetermined threshold.

Alternatively, or in addition, a user may override the machine learning model output via user input 532 when manual mode is selected on mode input 534. In this case the stimulus generator 520 is controlled directly by the user and the user input data is combined with the sensor data received on the processor input 502 by the training data compiler 540. This additional data may be used to run a further training cycle on the machine learning models 510a, 510b, 510c to generate a further adapted machine learning model which may be stored in memory (not shown) for use when the audio controller is subsequently activated. In this way the audio controller 500 may be adapted to automatically adjust the auditory signal characteristics for a specific user to alleviate motion sickness dependent on the context.

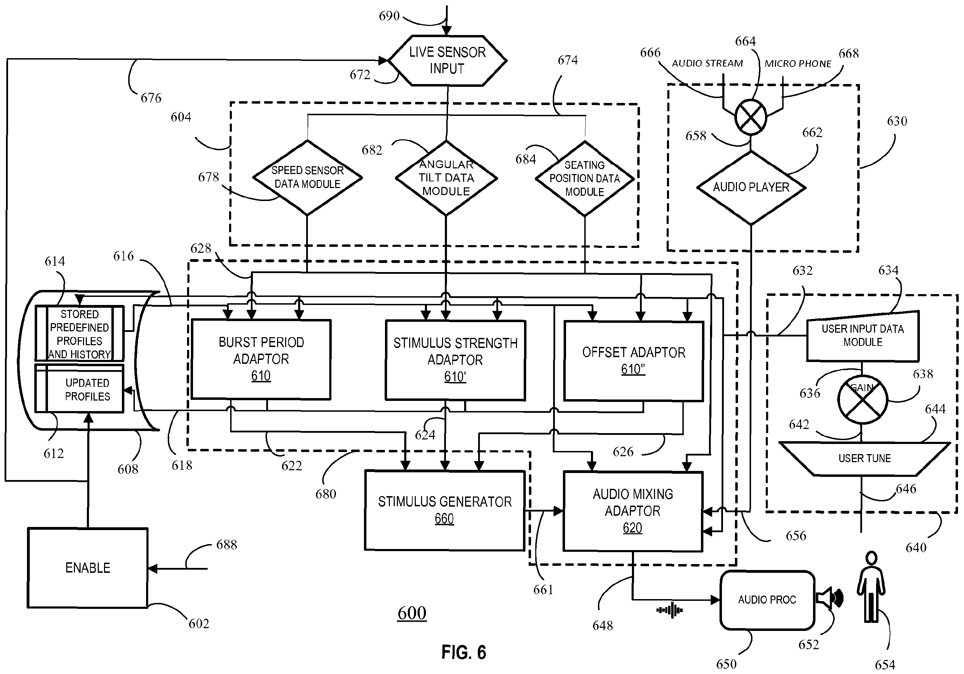

Figure 6 shows an audio controller 600 for use in a vehicle. Audio controller 600 includes a sensor input module 604, a processor 680 which may be referred to as an artificial intelligence (AI) processor, a memory 608, an enable module 602, an audio input module 630, a user interface module 640, an audio processor 650, a stimulus generator 660, and a sensor input interface 672.

The enable module 602 may enable or disable the audio controller 600 determined from a control signal from the enable input 688. In some examples the enable input may be directly controlled by the user. The enable module 602 may have an output 676 connected to the memory 608 and a sensor input interface 672. The sensor input module 604 which may also be referred to as a sensor fusion module may have a speed sensor data module 678, angular tilt data module 682, and seating position data module 684.

The sensor input interface 672 may have a sensor input 690 connected to one or more different sensors (not shown). The connection to the sensors may be wired and/or wireless. The sensor input interface 672 may have an output 674 connected to a speed sensor data module 678, angular tilt data module 682, and seating position data module 684. The sensor input module 604 may have an output 628 connected to an output of the speed sensor data module 678, angular tilt data module 682, and seating position data module 684. In other examples different sensor input data modules may be used.

The audio input module 630 may include a mixer 664 and an audio player 662. The audio input module may have an audio stream input 666 connected to a first input of the mixer 664 and a microphone input 668 connected to the second input of the mixer 664. The mixer output 658 may be connected to an input of the audio player 662. The output of the audio player may be connected to the audio input module output 656.

The user input module 640 may include a user tuning stage 644 having an input connected to the input 646 of the user input module 640. The user tuning stage output 642 may be connected to the input of a gain stage 638. The gain stage output 636 may be connected to an input of the user input data module 634. The output of the user input data module 634 may be connected to the user input module output 632. The user input module output 632 may be connected to an input of the memory 608.

The AI processor 680 may include a burst period adaptor 610, a stimulus strength adaptor 610', an offset adaptor 610", and an audio mixing adaptor 620. Each of the burst period adaptor 610, stimulus strength adaptor 610', offset adaptor 610", and audio mixing adaptor 620 may include a machine learning model. Each of the burst period adaptor 610, stimulus strength adaptor 610', offset adaptor 610", and audio mixing adaptor 620 may have a first input connected to an output 616 of the memory 608. Each of the burst period adaptor 610, stimulus strength adaptor 610', offset adaptor 610", and audio mixing adaptor 620 may have a second input connected to the sensor input module output 628. Each of the burst period adaptor 610, stimulus strength adaptor 610', offset adaptor 610", and audio mixing adaptor 620 may have a third input connected to the user input module output 632. The audio mixing adaptor 620 may have a fourth input connected to the audio input module output 656. The audio mixing adaptor 620 may have a fifth input connected to the stimulus generator output 661.

The audio mixing adaptor 620 may have an output 648 connected to an input of the audio processor 650. The audio processor output may be connected to an acoustic transducer such as a loudspeaker 652. Each of the burst period adaptor 610, stimulus strength adaptor 610', offset adaptor 610", and audio mixing adaptor 620 may have a first output connected to a second input 618 of the memory 608. The burst period adaptor 610 may have burst control output 622 connected to a first input of the stimulus generator 660. The stimulus strength adaptor 610 may have stimulus strength control output 624 connected to second input of the stimulus generator 660. The offset adaptor 610 may have an offset control output 626 connected to a third input of the stimulus generator 660.

The audio controller 600 makes use of three types of sensor data from the sensor input module 604 to adapt a stimulus generated to alleviate motion sickness and optionally mix with a further adapted audio input signal. The types of sensor data may provide information on 1) Increase or Decrease in Speed of the vehicle, 2) Angular tilt of the car based on the road conditions (hilly or straight roads), and 3) Seating Position inside the car (i.e. if the person is seated facing to the direction of driving, seated in front or behind, seating in the direction opposite to the driving direction). In other examples, other types of sensor data may also be used. In some examples fewer or more than three types of sensor data may be used.

The operation of the audio controller 600 is now described assuming the audio controller 600 and the user is located in a vehicle such as a car. The audio controller 600 may be part of an in-car infotainment system or may be incorporated into a wearable device such as a hearable or smart watch.

The audio controller 600 may be enabled by the enable module602. This may be enabled directly by the user on entering the car or indirectly by detecting that the user is in a vehicle. The enable module 602 may enable the sensors directly (not shown). The enable module 602 may enable the sensor input interface 672 and the sensor input 690 to allow sensor information to be received for example from a wireless communication link. The AI processor 680 may retrieve the stored predefined profiles 614 from the memory 608 and history to be read as initial training data for configuring the machine learning models in each of the burst period adaptor 610, stimulus strength adaptor 610' and offset adaptor 610". The selection of the profile from memory 608 may be determined directly by a user 654 controlling the user input module 640 or from initial sensor data received via the sensor input interface 672 and output on the sensor input module output 628.

Once configured, the Al processor 680 may control the stimulus generator 660 based on the sensor input data received from the sensor input module 604 for generating the auditory stimulation.

Extracted information from the sensor input module 604 may be provided to the AI processor 680 for machine learning and training the parameters to control the stimulus generator in a similar way to that described for audio controller 500. In the AI processor 680 the sensor data may be stored to provide statistical information and to adapt the response of the burst period adaptor 610, the stimulus strength adaptor 610' and the offset adaptor 610". The auditory stimulation generated by the stimulus generator 660 may be adapted by adapting the burst period of tones in response to increase or decrease in speed. The auditory stimulation generated by the stimulus generator 660 may be adapted by strengthening the stimulation frequency dependent on the angular tilt of the car. The auditory stimulation generated by the stimulus generator 660 may be adapted by strengthening the stimulation frequency dependent on the angular tilt of the car. The auditory stimulation generated by the stimulus generator 660 may be adapted by adding an offset, corresponding to a minimum signal level to the stimulation dependent on seating position in the vehicle.

The user 654 may override the default control be each of the burst period adaptor, the stimulus strength adaptor and the offset adaptor. In this case, the desired response corresponding to the user input value may be captured together with the sensor input data to provide additional training data. The burst period adaptor 610, stimulus strength adaptor 610' and the offset adaptor 610" may switch to a further training cycle using the updated training data to further adapt the response so that in future, the auditory stimulation generated by the stimulus generator 660 more closely matches that desired by a particular user 654.

When the audio controller 600 is deactivated which may be for example in response to the vehicle being turned off, the enable module 602 may enable the memory 608 to receive the additional trained configuration data or updated profiles 612 from each of the burst period adaptor 610, the stimulus strength adaptor 610' and offset adaptor 610". These updated profiles 612 may be subsequently used when the audio controller is next activated.

The auditory stimuli which may be in the inaudible frequency range is provided as first input to the audio mixing adaptor 620.

An audio stream received on audio stream input 666 may be mixed by mixer 664 with a microphone signal received on microphone input 668. The mixed audio signal may be output on mixer output 658. The audio player 662 may format the signal and provide it to the audio mixing adaptor 620. The with the auditory stimulation which is generated by the stimulus generator 660 which may typically be in the inaudible frequency range. The audio mixing adaptor 620 may adapt the received audio signal to alter the frequency spectrum of the audio signal in addition to the stimulus generation in order to remove infrasound and other frequencies that may cause nausea, headaches or dizziness in some people.

In some examples the audio mixing adaptor 620 and the audio input module 630 may be removed.

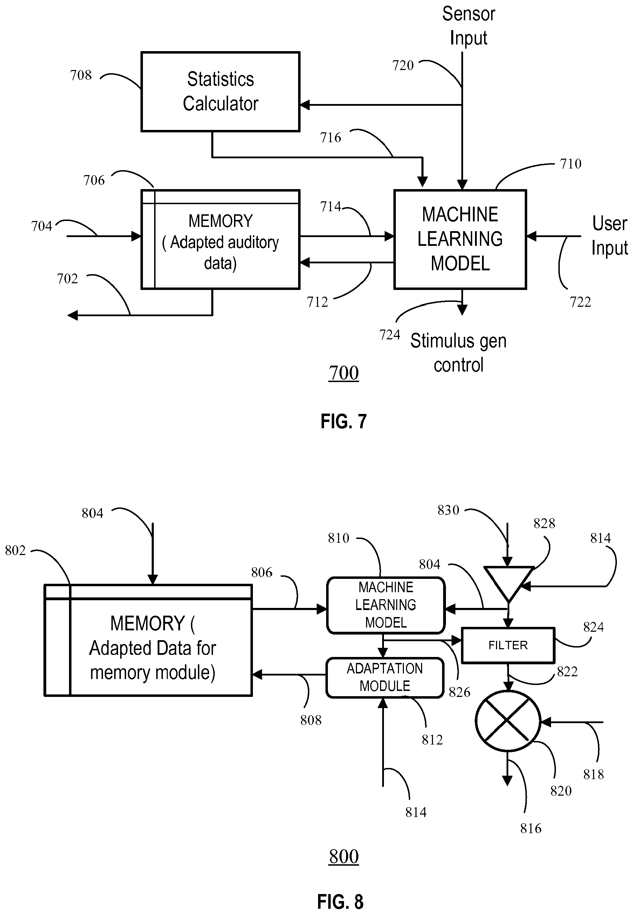

Figure 7 shows an example stimulus parameter adaptor 700 that may be used to implement the burst period adaptor 610, the stimulus strengthening adaptor 610' and the offset adaptor 610".

The stimulus parameter adaptor 700 has a machine learning model 710, a statistics calculator 708 and stimulus parameter adaptor memory 706. A user input 722 may be connected to the machine learning model 710. A sensor input 720 may be connected to the machine learning model 710 and the statistics calculator 708. The stimulus parameter adaptor memory 706 may have a first input 704 and first output 702 for communication with a further memory. Referring to the audio controller 600, this further memory may for example be the memory 608. The stimulus parameter adaptor memory 706 may have a second input 712 and second output 714 connected to the machine learning model 710. In operation the stimulus parameter adaptor 700 may receive initial configuration information from a further memory which is loaded into the stimulus parameter adaptor memory 706 via first input 704. This configuration information may be used to configure the machine learning model 710 via second output 714.

The machine learning model 710 may output a control signal for a stimulus generator via stimulus control output 724 dependent on the input data received. A user may override the default behaviour of the machine learning model 710 via user input 722. In this case the user input data and the sensor input data may be transferred to the stimulus parameter adaptor memory 706 via second input 712 and used as training data. The training data may subsequently be used to further train or adapt the machine learning model 710. Alternatively, or in addition, the machine learning model 710 may be directly adapted based on the output of the statistics calculator 708.

The statistics output 716 may adapt the threshold for the activation function in the layers of the neural network implementing the machine learning model 710 from a-priori rules on characteristics which may increase or reduce the likelihood of motion sickness. These may include rules for example to increase the stimulus level if the sensor data indicates frequent change of direction or variation of tilt of a vehicle above a predetermined threshold. If, for example, a change of direction happens more than X times in a given time period Y, then the threshold to stimulate may be reduced versus a default threshold setting. The default threshold setting may be the threshold which is used by the training data. If the change of direction happens much less than V times in a given time period W, the default threshold setting as stored in the memory is used.

In this way, the collected statistics may sensitise the neural network to increase the likelihood of stimulation.

Figure 8 shows an example audio mixing adaptor 800 that may be used to implement the audio mixing adaptor 620. An audio input 830 may be connected to a first amplifier or gain stage 828. The first gain stage 828 may have a control input connected to a user control input 814. The output 804 of the first gain stage 828 may be connected to a machine learning model 810 and an input of a filter 824 which may act as an adaptive filter. The filter output 822 may be connected to a first input of an audio mixer 820. The second input 818 of the audio mixer 820 may be connected to a stimulus generation input. When included in an audio controller, for example audio controller 600, this stimulus generation input may be connected to the output of a stimulus generator, for example stimulus generator 660. The mixer 820 has a mixer output 816.

The machine learning model 810 may have an output 826 connected to a control input of the filter 824. The output 826 may be connected to an input of the adaptation module 812. The adaptation module 812 may have a control input connected to the user input 814. An output 808 of the adaptation module 812 may be connected to an audio mixer memory 802. A first input 806 of the machine learning model 810 may be connected to an output of the audio mixer memory 802. A second input 804 of the audio mixer memory may be connected to a further memory (not shown).

In operation of the audio mixing adaptor 800, the audio mixer memory 802 may initially receive the environmental context setting from predefined profiles which may vary dependent for example on whether the user is in a car, a plane, or using a VR headset for gaming. These predefined profiles may be used to initially configure the machine learning model 810. An audio stream may be received on audio input 830. This audio stream may include a combination of audio data and microphone input data. The user may control the first gain stage 828 via user input 814 to add or reduce the gain strength for certain conditions.

The machine learning model 810 may receive the audio stream for feature extraction to determine environmental context. The machine learning model 810 may output a control signal for the filter 824 which may modify the audio stream based on the identified environmental context (e.g. filter settings, gain strengths). The machine learning model 810 may include one or more trained neural networks or machine learning models which select the most appropriate gain, and filter coefficients by classification in a similar way to described for the audio controller 500. The mixer 820 may overlay generated stimulus for alleviating motion sickness received on stimulus input 818 on top of the input audio stream received from the second gain stage output 822.

The adaptation module 812 may combine user feedback with the output of the machine learning model to provide additional training data for incremental learning. This training data may be transferred back to the audio mixer memory 802 and used to further adapt the machine learning model 810.

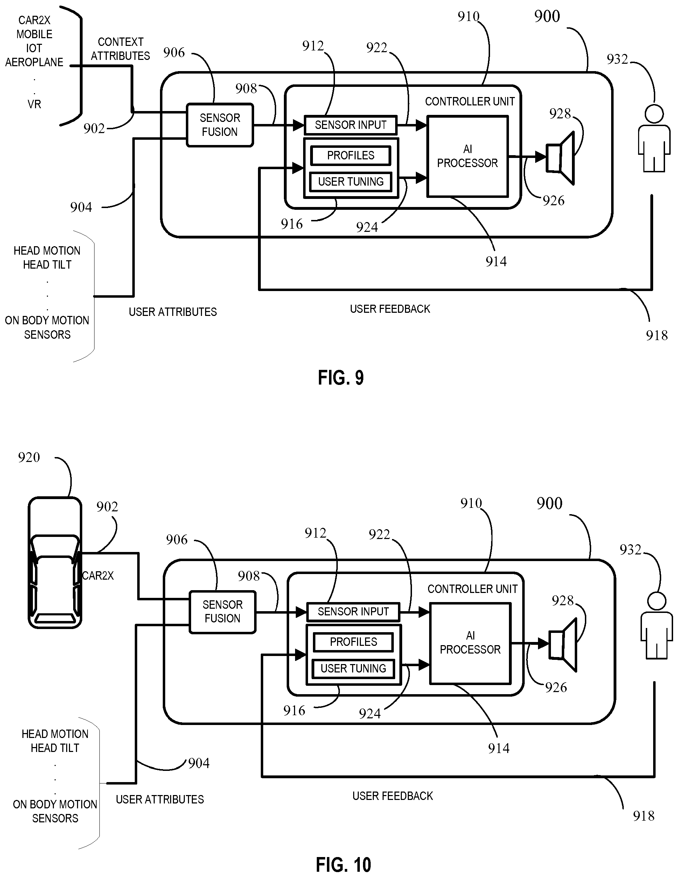

Figures 9 to 12 show an intelligently controlled auditory device 900 in various application use cases. The auditory device 900 may be incorporated into a hearable device or other hearing instrument. The auditory device 900 may include a sensor fusion module 906, a controller unit 910 including a memory 916, a sensor data module 912 and an AI processor 914. The sensor fusion module 906 may have a context attribute data input 902 for receiving context related information for example from a vehicle wireless connection, a mobile phone, Internet of Things (IoT) device, aircraft, or virtual reality headset. The sensor fusion module 906 may have a user attribute data input 904 for receiving user-related information for example on head motion, head tilt or other information from on-body motion sensors of a user of the auditory device. The sensor fusion module output 908 may be connected to sensor data module 912. In some examples the sensor fusion module 906 and the sensor data module 912 may be combined into a sensor data input module. The sensor data module output 922 may be connected to the AI processor 914. The memory 916 may have an output 924 connected to the AI processor 914. The memory 916 may have an input connected to a user feedback input 918. The AI processor 914 may be connected to an audio processor (not shown) which is connected to the controller unit output 926. Controller unit output 926 may be connected to a loud speaker 928.

In operation of the auditory device 900, sensors coupled to the sensor fusion module 906 detect context attribute data based on the application. The sensor information may be used to determine of the environment of the user 932, thereby it captures if a person 932 is sitting, driving, moving, etcetera. The sensors maybe wirelessly connected or integrated into the auditory device 900. For example, if the auditory device 900 is incorporated into a hearing aid or other hearable device, sensory information may be obtained from sensors present in the hearables as well as from sensors present in external sources such as mobile phone, vehicle wireless infrastructure, VR infrastructure or other means. A user may influence which sensors are to be used.

In particular, for sensory information from external sources, there is a data communication pipe needed between sensory functions and the Hearables and/or Hearing Instruments. This data pipe may be via a wireless communication means like Bluetooth RF or NFMI.

In other examples, sensor information could be provided by the engine control unit (ECU) or car infotainment system. This sensor information may include speed, angular movement, and direction of the car based on the Global Positioning System (GPS) data. This sensor data may be either broadcasted via ECU or made available to person on seats which then can be communicated via Body Area Network (BAN) or NFMI to the device. Hearable devices may include wireless communication to enable a duplex communication channel from the vehicle to hearable and optionally vice-versa. The auditory controller may use Flight details if the context is a plane, Gyro information for specific movements if the context is a ship, or frames per second (FPS) if the context is a VR headset.

The controller unit 910 may process the sensor information received and performs the sensor fusion algorithms which processed the sensor data at the same time, for example using sensor fusion module 906. The sensor input module 912 may perform filtering of the sensor data. The AI processor 914 may perform the spectrum shaping of an auditory stimulus and generate the adapted stimulus signal for alleviating motion sickness. With the Al processor 914 enabled and the context attribute data, the controller unit 910 may adapt the generated stimulus for the user requirements eventually as the scenario changes. Stimulation control can be personalized for each user independently.

Based on the different application environments, the auditory stimulation can be varied accordingly. For example, if a person is listening to music in an autonomous car, the Al processor 914 may adapt the smart filter- and spectrum shaping functions to enrich the frequency spectrum of the music by filtering infrasound and providing additional frequency content, which may be in the inaudible frequency range for mitigating motion illness symptoms. In Gaming or VR applications, controller unit 910 may receive information about the running video for example the frame per second or video frame rate. Based on the information. The AI processor 914 may adapt the auditory signals to match the video or game visualized. The auditory signals may alleviate VR sickness.

The auditory controller 900 may adapt the profile settings based on the user for different levels of imbalance. The AI processor 914 may use sensory inputs to learn the different aspects of the environmental conditions and supplements for the behavioural characteristics of a user. The auditory controller 900 may include predefined profiles to provide an initial stimulus. The user may adjust or change the threshold levels of stimulus based on individual requirements. The AI processor 914 may learn the kind of stimulus to be output from the user feedback for the different scenario's and eventually perform the action of the user automatically. The user may override the AI processor 914 for tuning at any point of user's choice. The behavioural qualities captured by the AI processor 914 may be stored as specific user profile settings in the memory 916 which may also be reapplied in other devices.

The auditory device 900 may be plugged in to the ear to stimulate the vestibular system based on sensory information automatically without manual intervention. The auditory device 900 may be incorporated in one of a pair of hearable devices such as ear buds or hearing aids. Synchronization between ear buds or hearing aid devices may be done by wireless communication for example using NFMI wireless communication technology.

Figure 10 shows the auditory device 900 in a vehicle context communicating with sensors in a vehicle 920 for example using a vehicle wireless communication link which may be an autonomous vehicle. Sensed properties in the vehicle wireless infrastructure includes among others, but not limited to, speed, inclination during travel, Gyro inputs, GPS information for the travel route (i.e. hilly region or straight roads), location of the user 932 seated in vehicle with the direction, elevation details. Based on the sensed properties controller unit 910 may stimulate the audio signals and with user feedback for the Al processor 914, the auditory device 900 is adapted. In this way, a profile can be customized to individual based on the conditions of travel, or operation mode.

The sensed properties may be input to controller unit 910 to provide the auditory stimulus as explained previously for figure 9. Controller unit 910 may be enabled to receive the sensed properties in a vehicle 920 by different methods. Infotainment systems in vehicle 920 may provide the details of all the properties required for the controller unit such as speed, distance travelled. Hearables devices including the controller unit 910 may receive the information broadcast using Vehicle wireless communication protocols. The sensed properties may be communicated in different ways, for example via seats including BAN technology for individual users.

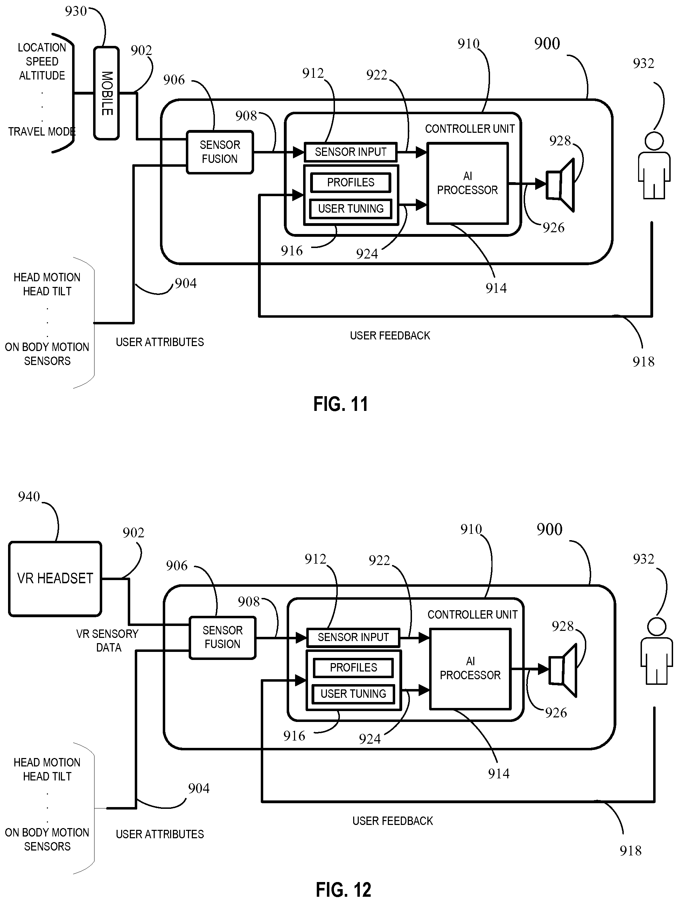

Figure 11 shows the auditory device 900 communicating with sensors in a mobile device 930. In this context, the user 932 makes use of a mobile device 930 combined with hearables and/or hearing Instruments. Sensors within the mobile device 930 may provide data on speed, altitude and location. In addition, the user may set different inputs concerning travelling related information such as, if travelling backwards (seat location), if travelling by car/bus/train/plane, or other settings that may be relevant settings. The mobile device 930 may store a number of predefined profiles, which can be customized depending on the user needs. The mobile device 930 may receive the context attribute information from external sources of sensors. The various sources may include the speed of the person travelling, location, altitude information, and travel mode. This sensed information may be provided to controller unit 910 from the mobile device 930 along with user attribute information via user attribute data input 904. In other examples a smart wrist watch may be used to collect sensory information, for example detecting speed, angular movement and enable to communicate with the auditory device via BAN or NFMI technology.

The controller unit 910 may include predefined profiles and user feedback or tuning inputs stored in memory 916. The AI processor 914 may stimulate the auditory signals dependent on the sensor inputs. During the initial phase of Al learning, predefined profiles provide the required inputs to trigger the stimulus. A user may provide feedback via the user feedback input 918 to tune the stimulus thresholds based on individual need. With Al processing enabled, the controller unit 910 may learn the kind of stimulus to be triggered for different properties from the user and adapt the output.

By enabling the feedback path from a user 932, Al processor 914 may adapt and control the stimulus automatically based on the sensed properties. In some examples, the user may save the profile settings customized to individual, so that it can be used in other devices.

Figure 11 shows the auditory device 900 used in a Gaming or VR context coupled to a VR headset 940. In this context, the user 932 is wearing the VR headset 940 and the controller unit 910 may receive the FPS, and VR sensory information about the running video. VR sensory information of a scene in a running video may include multiple types of information. The main sensors are typically magnetometers which give information on the direction facing on the surface of the earth, accelerometers and gyroscopes which provide details on the orientation. In addition, the rendering speed of the scene on the display may be provided which causes the retina movement without the head movements. Based on the sensed properties the Al processor 914 may adapt the generation of the auditory stimulus signals to match the dynamics in visualization.

An audio controller for generating a signal for alleviating motion sickness is described. The audio controller comprises a sensor input module, a user control input and a processor comprising a machine learning model corresponding to a desired audio stimulation profile. The machine learning model is coupled to the sensor input module and the user control input. The machine learning model is configured to receive a sensor signal comprising at least one user attribute and at least one context attribute from the sensor input module. The audio controller includes a stimulus generator coupled to the machine learning model. The machine learning model controls the stimulus generator to generate a reference signal for alleviating motion sickness and adapts the reference signal dependent on at least one of the user control input and the sensor signal.

It is to be understood that many modifications can be made to the present disclosure and that the scope of the present invention is defined by the appended claims.

**TABLE OF REFERENCE SIGNS**

| | |
|---|---|
| 100 | audio controller |
| 102 | sensor input |
| 104 | user selection input |
| 106 | memory output |
| 108 | sensor output |
| 110 | sensor module |
| 112 | user control input |
| 114 | machine learning model first output |
| 116 | machine learning model second output |
| 118 | stimulus generator output |
| 120 | machine learning model |
| 122 | memory |
| 124 | stimulus generator |
| | |
| 200 | audio controller |
| 202 | sensor input |
| 204 | user selection input |
| 206 | memory output |
| 208 | sensor output |
| 210 | sensor module |
| 212 | user control input |
| 214 | machine learning model first output |
| 216 | machine learning model second output |
| 218 | stimulus generator output |
| 220 | machine learning model |
| 222 | memory |
| 224 | stimulus generator |
| 226 | audio mixer machine learning model output |
| 228 | audio input |
| 232 | Audio mixer |
| | |
| 234 | Audio mixer first audio output |
| 236 | Audio mixer machine learning model |
| 238 | Audio mixer machine learning model output |
| | |
| 300 | Method of audio signal generation to alleviate motion sickness |
| 302 | ICAD activation step |
| 304 | Machine model load step |
| 306 | Machine model adaptation |
| 308 | Auditory stimulation control |
| 310 | Deactivation check |
| 312 | end |
| | |
| 400 | Method of audio signal generation to alleviate motion sickness |
| 402 | ICAD activation check |
| 404 | Default mode check |
| 406 | Default profile load |
| 408 | Adapted profile load |
| 410 | Training mode check |
| 412 | Control stimulus |
| 414 | Context, user input data capture |
| 416 | Training data update |
| 418 | Training cycle execution |
| 422 | Profile update check |
| 424 | Profile update |
| 426 | Control stimulus |
| 428 | Check deactivation |
| 430 | Profile update |
| 432 | End process |
| | |
| 500 | AI processor |
| 502 | Processor input |
| 504a | Neural network input layer |
| 504b | Neural network input layer |
| 504c | Neural network input layer |
| 506a | Neural network hidden layer |
| 506b | Neural network hidden layer |
| 506c | Neural network hidden layer |
| 508a | Neural network output layer |
| 508b | Neural network output layer |
| 508c | Neural network output layer |
| 510a | machine learning model |
| 510b | machine learning model |
| 510c | machine learning model |
| 512a | machine learning model output |
| 512b | machine learning model output |
| 512c | machine learning model output |
| 514 | Gain controller |
| 516 | Frequency controller |
| 518 | Offset controller |
| 520 | Stimulus generator |
| 522 | Gain controller output |
| 524 | Frequency controller output |
| 526 | Offset controller output |
| 528 | Stimulus output |
| 530 | Sensor statistics calculator |
| 532 | User input |
| 534 | Mode select input |
| 536 | User control output |
| 538 | Training data output |
| 540 | Training data compiler |
| 542 | Sensor statistics calculator output |
| | |
| 600 | Audio controller |
| 602 | Enable module |
| 604 | Sensor input module |
| 608 | memory |
| 610 | Burst period adaptor |
| 610' | Stimulus strength adaptor |
| 610" | Offset adaptor |
| 612 | Updated profiles |
| 614 | Predefined profiles |
| 616 | Memory first output |
| 618 | Memory second input |
| 620 | Audio mixing adaptor |
| 622 | Burst control output |
| 624 | Strength control output |
| 626 | Offset control output |
| 628 | Sensor input module output |
| 630 | Audio input module |
| 632 | User input module output |
| 634 | User input data module |
| 636 | Gain stage output |
| 638 | Gain stage |
| 640 | User interface module |
| 642 | User tuning stage output |
| 644 | User tuning stage |
| 646 | User input module input |
| 648 | Audio mixing adaptor output |
| 650 | Audio processor |
| 652 | Loudspeaker |
| 654 | user |
| 656 | Audio input module output |
| 658 | Mixer output |
| 660 | Stimulus generator |
| 661 | Stimulus generator output |
| 662 | Audio player |
| 664 | Audio player mixer |
| 666 | Audio stream input |
| 668 | Microphone input |
| 672 | sensor input interface |
| 674 | Sensor input interface output |
| 676 | Enable module output |
| 678 | Speed sensor data module |
| 680 | processor |
| 682 | Angular tilt data module |
| 684 | Seating position data module |
| 688 | Enable input |
| 690 | Sensor input |
| | |
| 700 | Stimulus parameter adaptor |
| 702 | Memory first output |
| 704 | Memory first input |
| 706 | Adaptor memory |
| 708 | Statistics calculator |
| 710 | Machine learning model |
| 712 | Memory second input |
| 714 | Memory second output |
| 716 | Statistics output |
| 720 | Sensor input |
| 722 | User input |
| 724 | Stimulus control output |
| | |
| 800 | Audio mixing adaptor |
| 802 | Audio mixer memory |
| 804 | Audio mixer memory second input |
| 806 | First machine learning model input |
| 808 | Adaptation module output |
| 810 | Machine learning model |
| 812 | Adaptation module |
| 814 | User input |
| 816 | Mixer output |
| 818 | Stimulus input |
| 820 | Audio mixer |
| 822 | Filter |
| 824 | Filter output |
| 826 | Machine learning model output |
| 828 | Gain stage |
| 830 | Audio input |
| | |
| 900 | intelligently controlled auditory device |
| 902 | Context attribute data input |
| 904 | User attribute data input |
| 906 | Sensor fusion module |
| 908 | Sensor fusion module output |
| 910 | Controller unit |
| 912 | Sensor data module |
| 914 | AI processor |
| 916 | Memory |
| 918 | User feedback input |
| 920 | Vehicle |
| 922 | Sensor data module output |
| 924 | Memory output |
| 926 | Controller unit output |
| 928 | Loudspeaker |
| 930 | Mobile device |
| 932 | User |
| 940 | VR headset |

## Claims

1. An audio controller for generating a signal for alleviating motion sickness, the audio controller comprising:
a sensor input module;
a user control input;
a processor comprising a machine learning model corresponding to a desired audio stimulation profile, the machine learning model being coupled to the sensor input module and the user control input and configured to receive a sensor signal comprising at least one user attribute and at least one context attribute from the sensor input module;
a stimulus generator coupled to the machine learning model; and
wherein the machine learning model is configured to control the stimulus generator to generate a reference signal for alleviating motion sickness, the reference signal being adapted dependent on at least one of the user control input, and the sensor signal.

2. The audio controller of claim 1 wherein the machine learning model comprises a neural network and the processor is configured to adapt the machine learning model by modifying the activation threshold of layers of the neural network dependent on the sensor signal.

3. The audio controller of claim 2 wherein the machine learning model comprises a first neural network configured to control the period between bursts of the audio stimulus, a second neural network configured to control the gain of the audio stimulus, and a third neural network configured to control the offset of the audio stimulus.

4. The audio controller of any preceding claim, wherein the processor is configured to compile additional training data dependent on the external user input and the sensor signal; to adapt the machine learning model in a training cycle using training data comprising the additional training data and to generate an audio stimulation profile comprising the adapted machine learning model.

5. The audio controller of claim 4 further comprising a memory coupled to the processor and configured to update the audio stimulation profiles in the memory with the adapted machine learning models.

6. The audio controller of any preceding claim further comprising
an audio input configured to receive an audio signal;
a mixer coupled to the audio input and the stimulus generator output;
wherein the processor comprises a further machine learning model corresponding to the desired audio stimulation profile coupled to the audio input and a filter;
the further machine learning model is configured to control the filter to adapt the audio signal to at least attenuate infrasound signals dependent on at least one of an external user input and the sensor signal; and
wherein the mixer is further configured to mix the adapted audio signal and the reference signal and to output the mixed adapted audio signal and the reference signal.

7. The audio controller of any preceding claim wherein the desired audio stimulation profile is determined from at least one of a user control input and the sensor signal.

8. The audio controller of any preceding claim wherein the sensor input module is configured to receive a video frame rate from a video device and wherein the at least one context attribute comprises the video frame rate.

9. The audio controller of any of claims 1 to 7 wherein the sensor input module is configured to receive at least one of a tilt value and a user seating position value, and wherein the at least one context attribute comprises at least one of the speed of a vehicle, the tilt of a vehicle, and the user seating position and the at least one user attribute comprises at least one of head motion and head tilt.

10. A wearable device comprising the audio controller of any preceding claim and further comprising a wireless receiver coupled to the sensor input, wherein the wearable device is configured to receive context attributes via the wireless receiver.

11. A computer program product comprising instructions which, when being executed by the processor of the audio controller according to any of claims 1-10, cause said processor to perform the steps of
receiving a sensor signal comprising at least one user attribute and at least one context attribute from a sensor input module;
providing the sensor signal and the at least one context attribute to a machine learning model;
controlling a stimulus generator using the machine learning model to generate a reference signal for alleviating motion sickness dependent on at least one of the adapted audio stimulation profile and the user input.

12. The computer program product of claim 11 wherein the machine learning model comprises a neural network and the computer program product further comprises instructions which, when being executed by the processor, cause the processor to perform the steps of adapting the machine learning model by modifying the activation threshold of layers of the neural network dependent on the sensor signal.

13. The computer program product of claim 12 further comprising instructions which, when being executed by the processor, cause the processor to perform the steps of controlling the period between bursts of the audio stimulus with a first neural network of the machine learning model, controlling the gain of the audio stimulus with a second neural network of the machine learning model, and controlling the offset of the audio stimulus with a third neural network of the machine learning model.

14. The computer program product of any of claims 11 to 13 further comprising instructions which, when being executed by the processor, cause the processor to perform the steps of compiling additional training data dependent on the external user input and the sensor signal; adapting the machine learning model in a training cycle using training data comprising the additional training data; and storing the adapted machine learning model.

15. The computer program product of any of claims 11 to 14 further comprising instructions which, when being executed by the processor, cause the processor to perform the steps of:
receiving an audio signal;
using a further machine learning model corresponding to the desired audio stimulation profile to control the filtering of the audio signal to at least attenuate infrasound signals dependent on at least one of the external user input and the sensor signal;
mixing the filtered audio signal and the reference signal.

## Patentansprüche

1. Audiosteuereinheit zum Erzeugen eines Signals zum Lindern einer Bewegungskrankheit, wobei die Audiosteuereinheit Folgendes umfasst:
ein Sensoreingangsmodul;
einen Anwendersteuereingang;
einen Prozessor, der ein Modell für maschinelles Lernen umfasst, das einem gewünschten Audiostimulationsprofil entspricht, wobei das Modell für maschinelles Lernen an das Sensoreingangsmodul und den Anwendersteuereingang gekoppelt ist und konfiguriert ist, ein Sensorsignal, das mindestens ein Anwendermerkmal und mindestens ein Kontextmerkmal umfasst, vom Sensoreingangsmodul zu empfangen; und
einen Reizgenerator, der an das Modell für maschinelles Lernen gekoppelt ist; wobei
das Modell für maschinelles Lernen konfiguriert ist, den Reizgenerator zu steuern, ein Bezugssignal zum Lindern einer Bewegungskrankheit zu erzeugen, wobei das Bezugssignal abhängig von der Anwendersteuereingabe und/oder dem Sensorsignal angepasst wird.

2. Audiosteuereinheit nach Anspruch 1, wobei das Modell für maschinelles Lernen ein neuronales Netz umfasst und der Prozessor konfiguriert ist, das Modell für maschinelles Lernen durch Modifizieren des Aktivierungsschwellenwerts von Schichten des neuronalen Netzes abhängig vom Sensorsignal anzupassen.

3. Audiosteuereinheit nach Anspruch 2, wobei das Modell für maschinelles Lernen ein erstes neuronales Netz, das konfiguriert ist, den Zeitraum zwischen Serien des Audioreizes zu steuern, ein zweites neuronales Netz, das konfiguriert ist, die Verstärkung des Audioreizes zu steuern, und ein drittes neuronales Netz, das konfiguriert ist, den Versatz des Audioreizes zu steuern, umfasst.

4. Audiosteuereinheit nach einem vorhergehenden Anspruch, wobei der Prozessor konfiguriert ist, abhängig von der externen Anwendereingabe und dem Sensorsignal zusätzliche Trainingsdaten zu erstellen; unter Verwendung von Trainingsdaten, die die zusätzlichen Trainingsdaten umfassen, das Modell für maschinelles Lernen in einem Trainingszyklus anzupassen und ein Audiostimulationsprofil zu erzeugen, das das angepasste Modell für maschinelles Lernen umfasst.

5. Audiosteuereinheit nach Anspruch 4, die ferner einen Speicher umfasst, der an den Prozessor gekoppelt ist und konfiguriert ist, die Audiostimulationsprofile im Speicher mit den angepassten Modellen für maschinelles Lernen zu aktualisieren.

6. Audiosteuereinheit nach einem vorhergehenden Anspruch, die ferner Folgendes umfasst:
einen Audioeingang, der konfiguriert ist, ein Audiosignal zu empfangen; und
einen Mischer, der an den Audioeingang und den Reizgeneratorausgang gekoppelt ist; wobei
der Prozessor ein weiteres Modell für maschinelles Lernen, das dem gewünschten Audiostimulationsprofil entspricht und an den Audioeingang und ein Filter gekoppelt ist, umfasst;
das weitere Modell für maschinelles Lernen konfiguriert ist, das Filter zu steuern, das Audiosignal anzupassen, um Infraschallsignale abhängig von einer externen Anwendereingabe und/oder dem Sensorsignal mindestens zu dämpfen; und
der Mischer ferner konfiguriert ist, das angepasste Audiosignal und das Bezugssignal zu mischen und das gemischte angepasste Audiosignal und das Bezugssignal auszugeben.

7. Audiosteuereinheit nach einem vorhergehenden Anspruch, wobei das gewünschte Audiostimulationsprofil aus einer Anwendersteuereingabe und/oder dem Sensorsignal bestimmt wird.

8. Audiosteuereinheit nach einem vorhergehenden Anspruch, wobei das Sensoreingangsmodul konfiguriert ist, eine Videobildrate von einer Videovorrichtung zu empfangen, und das mindestens eine Kontextmerkmal die Videobildrate umfasst.

9. Audiosteuereinheit nach einem der Ansprüche 1 bis 7, wobei das Sensoreingangsmodul konfiguriert ist, einen Neigungswert und/oder einen Anwendersitzpositionswert zu empfangen, und das mindestens eine Kontextmerkmal die Geschwindigkeit eines Fahrzeugs und/oder die Neigung eines Fahrzeugs und/oder die Anwendersitzposition umfasst und das mindestens eine Anwendermerkmal eine Kopfbewegung und/oder eine Kopfneigung umfasst.

10. Tragbare Vorrichtung, die die Audiosteuereinheit nach einem vorhergehenden Anspruch umfasst und ferner einen Drahtlosempfänger umfasst, der an den Sensoreingang gekoppelt ist, wobei die tragbare Vorrichtung konfiguriert ist, Kontextmerkmale mittels des Drahtlosempfängers zu empfangen.

11. Computerprogrammprodukt, das Befehle umfasst, die, wenn sie durch den Prozessor der Audiosteuereinheit nach einem der Ansprüche 1-10 ausgeführt werden, den Prozessor veranlassen, die folgenden Schritte durchzuführen:
Empfangen eines Sensorsignals, das mindestens ein Anwendermerkmal und mindestens ein Kontextmerkmal umfasst, von einem Sensoreingangsmodul;
Bereitstellen des Sensorsignals und des mindestens einen Kontextmerkmals zu einem Modell für maschinelles Lernen und
Steuern eines Reizgenerators unter Verwendung des Modells für maschinelles Lernen, um ein Bezugssignal zum Lindern einer Bewegungskrankheit abhängig von dem angepassten Audiostimulationsprofil und/oder der Anwendereingabe zu erzeugen.

12. Computerprogrammprodukt nach Anspruch 11, wobei das Modell für maschinelles Lernen ein neuronales Netz umfasst und das Computerprogrammprodukt ferner Befehle umfasst, die, wenn sie durch den Prozessor ausgeführt werden, den Prozessor veranlassen, die Schritte des Anpassens des Modells für maschinelles Lernen durch Modifizieren des Aktivierungsschwellenwerts von Schichten des neuronalen Netzes abhängig vom Sensorsignal durchzuführen.

13. Computerprogrammprodukt nach Anspruch 12, das ferner Befehle umfasst, die, wenn sie durch den Prozessor ausgeführt werden, den Prozessor veranlassen, die Schritte des Steuerns des Zeitraums zwischen Serien des Audioreizes mit einem ersten neuronalen Netz des Modells für maschinelles Lernen, Steuerns der Verstärkung des Audioreizes mit einem zweiten neuronalen Netz des Modells für maschinelles Lernen und Steuerns des Versatzes des Audioreizes mit einem dritten neuronalen Netz des Modells für maschinelles Lernen durchzuführen.

14. Computerprogrammprodukt nach einem der Ansprüche 11 bis 13, das ferner Befehle umfasst, die, wenn sie durch den Prozessor ausgeführt werden, den Prozessor veranlassen, die Schritte des Erstellens von zusätzlichen Trainingsdaten abhängig von der externen Anwendereingabe und dem Sensorsignal; Anpassens des Modells für maschinelles Lernen in einem Trainingszyklus unter Verwendung von Trainingsdaten, die die zusätzlichen Trainingsdaten umfassen; und Speicherns des angepassten Modells für maschinelles Lernen durchzuführen.

15. Computerprogrammprodukt nach einem der Ansprüche 11 bis 14, das ferner Befehle umfasst, die, wenn sie durch den Prozessor ausgeführt werden, den Prozessor veranlassen, die folgenden Schritte durchzuführen:
Empfangen eines Audiosignals;
Verwenden eines weiteren Modells für maschinelles Lernen, das dem gewünschten Audiostimulationsprofil entspricht, um das Filtern des Audiosignals zu steuern, Infraschallsignale abhängig von der externen Anwendereingabe und/oder dem Sensorsignal mindestens zu dämpfen; und
Mischen des gefilterten Audiosignals und des Bezugssignals.

## Revendications

1. Organe de commande audio servant à générer un signal destiné à atténuer le mal des transports, l'organe de commande audio comportant :
un module d'entrée de capteur ;
une entrée de commande d'utilisateur ;
un processeur comportant un modèle d'apprentissage automatique correspondant à un profil souhaité de stimulation audio, le modèle d'apprentissage automatique étant couplé au module d'entrée de capteur et à l'entrée de commande d'utilisateur et configuré pour recevoir un signal de capteur comportant au moins un attribut d'utilisateur et au moins un attribut de contexte en provenance du module d'entrée de capteur ;
un générateur de stimuli couplé au modèle d'apprentissage automatique ; et
le modèle d'apprentissage automatique étant configuré pour commander le générateur de stimuli afin de générer un signal de référence destiné à atténuer le mal des transports, le signal de référence étant adapté en fonction de l'entrée de commande d'utilisateur et/ou du signal de capteur.

2. Organe de commande audio selon la revendication 1, le modèle d'apprentissage automatique comportant un réseau neuronal et le processeur étant configuré pour adapter le modèle d'apprentissage automatique en modifiant le seuil d'activation de couches du réseau neuronal en fonction du signal de capteur.

3. Organe de commande audio selon la revendication 2, le modèle d'apprentissage automatique comportant un premier réseau neuronal configuré pour commander la période entre des salves du stimulus audio, un deuxième réseau neuronal configuré pour commander le gain du stimulus audio, et un troisième réseau neuronal configuré pour commander le décalage du stimulus audio.

4. Organe de commande audio selon l'une quelconque des revendications précédentes, le processeur étant configuré pour compiler des données d'apprentissage supplémentaires en fonction de l'entrée d'utilisateur externe et du signal de capteur ; pour adapter le modèle d'apprentissage automatique dans un cycle d'apprentissage utilisant des données d'apprentissage comportant les données d'apprentissage supplémentaires et pour générer un profil de stimulation audio comportant le modèle d'apprentissage automatique adapté.

5. Organe de commande audio selon la revendication 4 comportant en outre une mémoire couplée au processeur et configurée pour mettre à jour les profils de stimulation audio dans la mémoire avec les modèles d'apprentissage automatique adaptés.

6. Organe de commande audio selon l'une quelconque des revendications précédentes, comportant en outre
une entrée audio configurée pour recevoir un signal audio ;
un mélangeur couplé à l'entrée audio et à la sortie du générateur de stimuli ;
le processeur comportant un autre modèle d'apprentissage automatique correspondant au profil souhaité de stimulation audio, couplé à l'entrée audio et à un filtre ;
l'autre modèle d'apprentissage automatique étant configuré pour commander le filtre afin d'adapter le signal audio pour au moins atténuer des signaux infrasonores en fonction d'une entrée d'utilisateur externe et/ou du signal de capteur ; et
le mélangeur étant en outre configuré pour mélanger le signal audio adapté et le signal de référence et pour délivrer le signal audio adapté mélangé et le signal de référence.

7. Organe de commande audio selon l'une quelconque des revendications précédentes, le profil souhaité de stimulation audio étant déterminé à partir d'une entrée de commande d'utilisateur et/ou du signal de capteur.

8. Organe de commande audio selon l'une quelconque des revendications précédentes, le module d'entrée de capteur étant configuré pour recevoir une fréquence d'images vidéo en provenance d'un dispositif vidéo et l'attribut ou les attributs de contexte comportant la fréquence d'images vidéo.

9. Organe de commande audio selon l'une quelconque des revendications 1 à 7, le module d'entrée de capteur étant configuré pour recevoir une valeur d'inclinaison et/ou une valeur de position assise d'utilisateur, et l'attribut ou les attributs de contexte comportant au moins un attribut parmi la vitesse d'un véhicule, l'inclinaison d'un véhicule et la position assise d'utilisateur, et l'attribut ou les attributs d'utilisateur comportant au moins un attribut parmi un mouvement de la tête et une inclinaison de la tête.

10. Dispositif vestimentaire comportant l'organe de commande audio selon l'une quelconque des revendications précédentes et comportant en outre un récepteur sans fil couplé à l'entrée de capteur, le dispositif vestimentaire étant configuré pour recevoir des attributs de contexte par l'intermédiaire du récepteur sans fil.

11. Produit de programme d'ordinateur comportant des instructions qui, lorsqu'elles sont exécutées par le processeur de l'organe de commande audio selon l'une quelconque des revendications 1 à 10, amènent ledit processeur à réaliser les étapes consistant à :
recevoir un signal de capteur comportant au moins un attribut d'utilisateur et au moins un attribut de contexte en provenance d'un module d'entrée de capteur ;
fournir le signal de capteur et l'attribut ou les attributs de contexte à un modèle d'apprentissage automatique ;
commander un générateur de stimuli à l'aide du modèle d'apprentissage automatique pour générer un signal de référence destiné à atténuer le mal des transports en fonction du profil de stimulation audio adapté et/ou de l'entrée d'utilisateur.

12. Produit de programme d'ordinateur selon la revendication 11, le modèle d'apprentissage automatique comportant un réseau neuronal et le produit de programme d'ordinateur comportant en outre des instructions qui, lorsqu'elles sont exécutées par le processeur, amènent le processeur à réaliser les étapes consistant à adapter le modèle d'apprentissage automatique en modifiant le seuil d'activation de couches du réseau neuronal en fonction du signal de capteur.

13. Produit de programme d'ordinateur selon la revendication 12, comportant en outre des instructions qui, lorsqu'elles sont exécutées par le processeur, amènent le processeur à réaliser les étapes consistant à commander la période entre des salves du stimulus audio à l'aide d'un premier réseau neuronal du modèle d'apprentissage automatique, à commander le gain du stimulus audio à l'aide d'un deuxième réseau neuronal du modèle d'apprentissage automatique, et à commander le décalage du stimulus audio à l'aide d'un troisième réseau neuronal du modèle d'apprentissage automatique.

14. Produit de programme d'ordinateur selon l'une quelconque des revendications 11 à 13, comportant en outre des instructions qui, lorsqu'elles sont exécutées par le processeur, amènent le processeur à réaliser les étapes consistant à compiler des données d'apprentissage supplémentaires en fonction de l'entrée d'utilisateur externe et du signal de capteur ; à adapter le modèle d'apprentissage automatique dans un cycle d'apprentissage utilisant des données d'apprentissage comportant les données d'apprentissage supplémentaires ; et à stocker le modèle d'apprentissage automatique adapté.

15. Produit de programme d'ordinateur selon l'une quelconque des revendications 11 à 14, comportant en outre des instructions qui, lorsqu'elles sont exécutées par le processeur, amènent le processeur à réaliser les étapes consistant à :
recevoir un signal audio ;
utiliser un autre modèle d'apprentissage automatique correspondant au profil souhaité de stimulation audio afin de commander le filtrage du signal audio pour au moins atténuer des signaux infrasonores en fonction de l'entrée d'utilisateur externe et/ou du signal de capteur ;
mélanger le signal audio filtré et le signal de référence.
